# EUROPEAN PATENT APPLICATION

(11) **EP 1 617 225 A1**
(43) Date of publication of application: **18.01.2006**
(21) Application number: 05015472.3
(22) Date of filing: 15.07.2005
(51) Int. Cl.: G01N 33/94, G01N 33/50

(54) **Method of determining optimal amounts of vasodilators in transdermal drug delivery formulations**

(30) Priority: 16.07.2004 US 893733
(71) Applicant: BioChemics, Inc., Danvers, Massachusetts 01923 (US)
(72) Inventor: Carter, Stephen G., Andover, Massachusetts (US); Zhu, Zhen, Tewksbury, Massachusetts 01876 (US); Patel, Kanu, Derry, New Hampshire 03038 (US)
(74) Representative: Fuchs Mehler Weiss & Fritzsche

(57) **Abstract**

A method for determining and demonstrating the role of vasodilator chemical agents in the development and practice of transdermal drug delivery systems. Vasodilator chemicals applied topically dilate the blood vessels in the skin tissue, which have been shown to facilitate or inhibit systemic or skin tissue deposition of drug substances. The level of stimulation and/or inhibition has been found to be dependent on the concentration and the identity of the specific vasodilator chemical(s) used as well as the drug molecule(s) to be delivered. This work teaches the need to consider specific formulation requirements when dealing with vasodilator chemicals for the creation of successful delivery vehicles in the transdermal drug delivery system. These requirements for very low concentrations of vasodilators were an unexpected and a surprise finding, in contrast to the concentrations of the vasodilators typically used to elicit an increase in skin blood flow.

## Description

### Background of the Invention

Different technologies have been previously developed and employed to deliver a variety of drugs through the skin for systemic distribution throughout the body. These transdermal technologies including patches, liposomes, iontophoresis, and sono-/phonophoresis have achieved limited success as useful drug delivery methods.

Patches are limited by the types of drugs that may be successfully delivered in sufficient quantities and speed to be clinically useful. A list of patch-compatible drugs includes: nicotine, estrogen, testosterone, fentanyl, nitroglycerin, and scopolamine. These drugs are capable of penetrating the skin when held in close and constant contact with skin in part as a result of their unique physicochemical characteristics. Liposomes, which are a complex and multifaceted technology designed in general to encapsulated or incorporate drug molecules to make them more compatible and therefore better penetrating through the stratum corneum. However, there are limitations to this technology with respect to the types of drugs that can be delivered transdermally and have been found to be typically less effective than patches for systemic transdermal drug delivery. Liposomal technology has shifted the application focus to a role in the tissue-specific delivery applications for drugs that have been injected intravenously, in particular in the field of oncology. Iontophoresis and phonophoresis have excellent utility regarding the ability to deliver wide varieties and classes of drug molecules. These technologies are still limited in their general usage, however, due to the need for an external device or apparatus to power the drug delivery and also the need for relatively long time periods to deliver a single dose of drug, requiring the patient to remain attached to the device during this time.

The goal of finding a widely applicable transdermal drug delivery system continues to be desirable for many drugs including those adversely affecting the gastrointestinal system and those drugs having a lower than optimal bioavailability index when taken orally. Also, for the advantage of avoiding the act of injecting or orally administering a drug, to improve the safety or the efficacy profiles of the therapeutic agent. The introduction of drug molecules into the skin tissue in clinically effective concentrations has been enhanced over the years through the incorporation of various chemical agents. These penetration-enhancing agents, designed to promote penetration through the stratum corneum, include various natural and synthetic lipids or lipid-like molecules or lipid-related molecules, or with the incorporation of different organic molecules into the drug delivery vehicle designed to disrupt the architecture of the skin or to physically remove the barriers of the skin. The goal of each of these applications has been to enhance the penetration of drug molecules deeper into the skin tissue, which in turn would hopefully assist in the uptake of the drug into the bloodstream. Despite advances in penetration chemistry and formulation improvements, the efficacy of total drug transportation from the skin into the bloodstream has not attained the needed bioavailability index to be clinically relevant.

Apparatus-driven transdermal delivery technologies, including iontophoresis and sono-/phonophoresis, use either mild electrical current or ultrasonic energy to physically drive the drug molecules into the skin and eventually into the bloodstream. These technologies have an ability to move broad classes and molecular sizes of drug molecules through the skin and into the bloodstream. Despite the successes, there remain limitations associated with these efforts, including the relatively long periods of time required to deliver a complete dose of the drug and the issue of needing patients attached to an apparatus to power the delivery for either of these techniques.

Limitations associated with the apparatus-free penetration enhancing technologies spurred the development of derivatives of these technologies in attempts to improve the bioavailability of the transdermally applied drugs. In addition to the further developments surrounding novel penetration enhancing molecules and systems, there were also some unique hybrid technologies that were investigated including the use of vasomodulatory molecules in combination with iontophoretic or phonophoretic apparatus.

Sage et al., U.S. Patent No. 5,302,172, previously described the advantages of introducing vasodilators to transdermally deliver an active drug molecule using iontophoresis to improve the delivery efficiency for the drug into the bloodstream. Masiz, U.S. Patent No. 5,460,821, also described improvements to the penetration enhanced transdermal delivery vehicles through the incorporation of chemical vasodilators into the delivery vehicle. Riviere, U.S. Patent No. 5,620,416, described the use of vasoconstrictors in combination with systemically delivered, orally administered drugs in order to concentrate the drugs in a local tissue (e.g., skin tumors) following application of the vasoconstrictors.

### Summary of the Invention

The present invention relates to a composition effective for delivering specific drug through the skin and into the bloodstream, and more particularly to a composition effective for delivering a specific drug through the outer layers of skin and into the surrounding skin tissue. The composition includes a complex mixture of substances designed to facilitate the penetration of the drug through the skin tissue as a function of specific penetration enhancing chemicals, optionally in combination with chemical agents that have the potential to elicit a vasodilator reaction in the capillaries as well as other blood vessels. The concentration of vasodilator chemicals is in a low concentration range. Limited concentration ranges of the vasodilator chemicals are identified and dictated as a function of the other penetration enhancing molecules, including the active drug.

Concentrations of the vasodilators in the skin tissue following the topical application have been found to be related to the concentration to elicit increased blood flow, however the concentration needed to obtain optimal transdermal transport of the drug molecule into the bloodstream and/or the skin tissue is typically considerably less than that required for optimizing blood flow.

The present invention also relates to the methods needed to obtain the optimal transdermal delivery of drugs as a function of enhanced delivery resulting from the presence of vasodilator chemicals relates to the topical application of therapeutic or diagnostic agents for the systemic or localized distribution of therapeutic or diagnostic agents for the purpose of treating or detecting diseases and medical conditions. The topically applied agents may be administered at different locations on the human body to achieve access to the circulatory system. The inclusion of chemical vasodilator facilitates the transportation of the therapeutic and diagnostic agents. Chemical vasodilators act through a mechanism-specific basis resulting in a stimulation or inhibition of the uptake of the therapeutic or diagnostic agents. The vasodilator chemical controls the specific mechanism involved in the blood vessel that is involved with the relaxation and dilation of the vessel. The relationship between the extent of dilation and the volume of blood flow through the vessel and the chemical mechanism involved with enhanced movement of drug molecules through the skin tissue, across the blood vessel wall and into the blood stream has been identified in this invention. Although the specific conditions will change for each specific chemical vasodilator, the methods employed to determine these conditions remain constant. The determination of the specific conditions required to optimize transdermal drug delivery with the use of a vasodilator requires the evaluation of various concentrations variations of the vasodilator, present in either a lipid-based vehicle in an in vivo setting to determine the effect on the drug delivery capabilities. The surprise finding described in this patent is that the relationship between vasodilator concentration, blood flow and drug transport from the skin tissue into the blood stream is typically not linked to the maximum vasodilator concentration. Instead, the vasodilator concentration that achieves the best drug delivery into the blood stream is at a fractional level of that required to achieve maximum blood flow into the skin tissue. Relationships between the physiology of enhanced blood flow, hydrodynamic pressure changes in the skin tissue and the uptake of drug molecules from the skin tissue into the blood stream are identified via experimental analysis. Assessment of the effect of specific vasodilators and the enhanced blood flow in the skin are performed using a Doppler blood flow monitor device, such as a laser Doppler perfusion imager. Transdermal delivery of the drug molecule as a function of the vasodilator is evaluated in a living suitable animal model or human test subject, topically applying the test formulations, which contain varying amounts of vasodilator. The amounts of vasodilator in the test formulations will typically be several orders of magnitude less than that concentration required to stimulate the blood flow in the skin. Effective transdermal drug delivery is measured by determining the amount of drug present in the blood plasma as a function of time following the single application. Typically the vasodilator concentration will be scaled to a level initiated at 10⁻⁴ x maximal blood flow concentration and then progressing to higher concentrations in factors of two.

The present invention is a surprise and unexpected finding as an elaboration and an expansion of novel understanding of the vascular basis of enhanced transdermal drug delivery for systemic or local skin tissue targeting of drugs, based loosely on the general principles described in the prior art. Previous findings and reports described the general enhancement of drug delivery with the co-administration of a vasodilator.

The new findings in this invention describe the methodology required to identify the vasodilator concentration for optimal transdermal drug delivery, which is typically several orders of magnitude lower than that required to stimulate a maximal transient increase in localized blood flow in the skin. In contrast to previous propositions and assumptions in the field, the presence of many vasodilators, in concentrations previously considered and used for transdermal drug delivery enhancement, may cause an inhibition of the transdermally applied drug into the plasma. As a result, the method described herein is necessary and essential for the development of subsequent systems for the transdermal delivery of drugs enhanced by the presence of vasodilator chemicals, either with the assistance of an apparatus or device (e.g., iontophoresis or needles) or if used in a chemical formulation alone.

This invention describes the need to topically apply vasodilators in a concentration range of exceedingly small levels, to interact with the skin's microvasculature to cause the maximal uptake of the drug into the bloodstream. This invention suggests the process of vasodilator enhanced uptake of drugs involves or requires previously unconsidered or identified processes, which utilize a specific, small dose-range dependent and therapeutic drug specific relationship to the enhanced transdermal uptake of the drugs.
- The introduction of vasodilators into different transdermal delivery systems has previously been described as a possible facilitator of the efficacy of transdermal drug delivery
- Previous attempts and applications of vasodilators in transdermal drug delivery vehicles and systems have not achieved uniform nor reproducible delivery profiles of drugs and therefore have not been clinically useful or acceptable
- Different vasodilators act through different physiological mechanisms to cause vasodilation
- Measurements of blood flow consequent to topical application of vasodilators is typically mono-phasic, achieving a maximal response with increasing amounts of vasodilator regardless of the physiological mechanism based on the action of the vasodilator.
- The mechanism of vasodilation-enhanced transportation of topically applied drugs is at least biphasic with respect to the vasodilator concentration.
- The doses of the vasodilator substance required for maximal stimulation or enhancement of the delivery of the therapeutic drug into the bloodstream is typically a concentration range that is several orders of magnitude less than previously considered in relationship to that concentration needed for enhanced blood flow.
- Exceeding the concentration range of the vasodilator that elicits the maximum uptake of the drug into the bloodstream may result in an inhibition of the enhanced uptake of the drug.
- The maximum concentration range of vasodilator chemical used to enhance the transdermal delivery of the drug molecule is different for each vasodilator and for each drug molecule and therefore needs to be empirically derived.
- The incorporation of specific vasodilator concentration ranges is also relevant for transdermal drug delivery vehicles that contain a reservoir and/or a patch-like device
- The incorporation of specific vasodilator concentration range is relevant for transdermal drug delivery vehicles that are composed of a reservoir and an external energy source device used for the application and delivery of the drug, such as with iontophoresis or sonophoresis
- The invention describes the need to evaluate and determine a range of optimum concentrations (e.g., 0.00001 to 2.0% w/w, preferably less than 1% w/w) of a chemical vasodilator in the drug formulation to maximize the efficiency of the transdermal delivery of the drug molecule.

### Detailed Description of the Invention

The present invention describes the method to develop a broadly applicable and useful transdermal drug delivery vehicle and delivery system that is enhanced by the presence of a chemical vasodilator in combination with other penetration enhancing substances. The present invention describes a method to develop an optimized transdermal drug delivery vehicle, which is in part based upon a surprise finding of a proposed mechanism utilizing chemically induced vasodilation but at ranges of vasodilator and vasodilation, which were previously not considered.

The combination of a vasodilator, with an active drug molecule, in a complex transdermal drug delivery vehicle, has been shown to improve the systemically circulating levels of a drug. The topical application of a vasodilator, either co-administered with the drug or independently administered before or after the application of the drug containing-vehicle, as a multiple step process, needs to consider the chemical and physical architecture of the skin tissue, the tissue dynamics of fluid transfer, and the physiological characteristics of the microvasculature and the larger vessels of the skin tissue when attempting to facilitate the transdermal drug delivery process. The action of the vasodilator as it functions to dilate the blood vessel needs to be assessed for each vasodilator not only as it promotes dilation and the subsequent increased blood flow and fluid leakage from the capillary and also with respect to the ability of the vasodilator to effect a maximum uptake of the drug from the skin into the bloodstream. The level of vasodilator needed to effect this transfer of drug from skin to blood has been typically found to be significantly less than that level required to effect maximum blood flow and dilation.

Vasodilators act by relaxing the smooth muscles in the walls of blood vessels in the body. Relaxation of blood vessels enables a larger volume of blood to pass through the vessel and into the tissue. The dilation of the blood vessels may be performed in a dose-dependent manner, with a typical plateau effect noted in the maximal dilation and blood flow corresponding to the highest doses of vasodilators used. While the dilation is a sigmoid or hyperbolic shaped curve with respect to the blood flow, the relationship to the uptake of drugs deposited into the skin tissue and moving into the bloodstream is not correlative directly to the measurement of blood flow. The relationship between vasodilator concentrations effect on the vascular network in the skin and the drug transportation into the bloodstream may be bi-phasic or tri-phasic. Biphasic referring to the dose dependent stimulation of drug uptake at the lower concentrations tested, typically at concentrations related to small increases in blood flow, according to Doppler laser blood flow measurements. The titration of increasing vasodilator concentrations is critical to obtain a maximum drug blood level, since as the vasodilator concentration is increased, it passes through an apex and then as the blood flow measurements are approaching maximum, the drug uptake decreases and is inhibited, even with respect to the level achieved in the control samples.

The concentration of the vasodilator used to achieve an optimal blood or plasma level is vasodilator-specific. Each vasodilator acts through a specific biochemical mechanism and elicits a vasodilatory effect at different concentrations with different kinetics or the dilation and the prolonged periods of dilation following initial exposure.

Previous work has demonstrated that the addition of a vasodilator seemed to be important to the improved efficiency of drug delivery in a transdermal drug delivery system. This current invention, further describes the critical and specific requirements within that previously described general phenomenon that was clearly not obvious for the development of a successful transdermal drug delivery system. The present invention describes a method required to identify specific mechanism-based processes that control the efficiency or total uptake of the drug, in the transdermal delivery that are dependent upon specific, narrow ranges of vasodilator. The system containing either too little or too much vasodilator, will yield a circulating level of drug that is greater than control values with only lipid or other penetration enhancing chemicals, however, still less than those levels desired to elicit a clinically significant result. Different drugs may require different vasodilators to generate an optimal circulating drug level following transdermal delivery. The concentration of the vasodilator must be carefully examined experimentally to determine the proper and necessary concentration to elicit the maximum drug uptake from the skin into the bloodstream.

The method in accordance with the preferred embodiment of the invention comprises determining the optimal amount of vasodilator in a topical formulation comprising the vasodilator and an active ingredient, said method comprising determining the concentration of vasodilator necessary to stimulate the maximal dermal blood flow, formulating a first topical formulation with a concentration of vasodilator that is 0.001 times the maximal blood flow concentration, formulating at least a second topical formulation with a vasodilator concentration greater than 0.001 times the maximal blood flow concentration but less than the maximal blood flow concentration, applying the formulations to the skin of an animal, preferably a human, and measuring the amount of the active ingredient present in the blood of the animal as a function of time. Preferably multiple formulations are prepared with varying amounts of vasodilator(s) within the aforementioned range, and the optical amount is arrived at by comparing the active amounts of active ingredient present in the blood or blood plasma. Preferably one of the formulations prepared has a vasodilator concentration that is less than the concentration required to stimulate blood flow in the animal. The maximal blood flow determination can be made using a laser Doppler perfusion imager.

The addition of the proper amount and species of vasodilator in combination with the active drug molecule can induce a mild or low level of dilation of the capillary blood vessels, which stimulates the uptake of the drug molecules from the skin into the bloodstream. In contrast the addition of either too little or too much vasodilator in the delivery vehicle, will either not induce sufficient capillary dilation resulting in a sub-optimum uptake of the drug or too much vasodilator will induce too great of an effect on the dilation of the vessel, causing an inhibition of the movement of the drug from the skin tissue into the blood.

Chemical vasodilators are defined as any chemical substances that can elicit the physiological response of dilating capillaries or other blood vessels. This works describes the methods that are required to be evaluated with respect to the vasodilators and the other components of the transdermal drug delivery vehicle. The finding described herein is the definition of precise concentrations and formulation requirements that must be present for transdermal drug delivery to be successful under these conditions.

A list of example vasodilators include but are not limited to: arginine, bencyclane fumarate, benzyl nicotinate, buphenine hydrochloride, ciclonicate, cyclandelate, ethyl nicotinate, hepronicate, hexyl nicotinate, hydralazine, inositol nicotinate, isoxsuprine hydrochloride, methyl nicotinate, minoxidol, naftidrofuryl oxalate, nicametate citrate, niceritrol, nicoboxil, nicofuranose, nicotinyl alcohol, nicotinyl alcohol tartrate, nitric oxide, nitroglycerin, nonivamide, oxpentifylline, papaverine, papaveroline, pentifylline, peroxynitrite, pinacidil, sodium nitroprusside, suloctidil, teasuprine, thymoxamine hydrochloride, tolazoline, vitamin E nicotinate, and xanthinol nicotinate. Centrally acting vasomodulatory agents include clonidine, quanaberz, and methyl dopa. Alpha-adrenoceptor blocking agents include indoramin, phenoxybenzamine, phentolamine, and prazosin. Adrenergic neuron blocking agents include bedmidine, debrisoquine, and guanethidine. ACE inhibitors include benazepril, captopril, cilazapril, enalapril, fosinopril, lisinopril, perindopril, quinapril, and ramipril. Ganglion-blocking agents include pentolinium and trimetaphan. Calcium channel blockers include amlodipine, diltiazem, felodipine, isradipine, nicardipine, nifedipine, nimodipine, and verapamil. Prostaglandins including: prostacyclin, thrombuxane A2, leukotrienes, PGA, PGA1, PGA2, PGE1, PGE2, PGD, PGG, and PGH. Angiotensin II analogs include saralasin.

The vasodilator species and concentration within the transdermal drug delivery formulation may be different for each drug and for each delivery requirement. There may be one or more vasodilator, acting in a similar or different mechanism within the same formulation. There may also be vasodilators that are added in tandem temporally or simultaneously to induce the optimal reaction and to create a tissue concentration profile of the vasodilators that optimizes the transdermal transportation of the drug into the tissue or the bloodstream. The vasodilator may serve exclusively as the vasodilation agent or it may also, in addition, serve other functions to the delivery complex such as to assist in the penetration of the active drug molecule or the penetration of the other components of the delivery vehicle, the vasodilator may also co-function by definition and by action as the active drug agent, or to a serve another undefined function to create the optimal chemistry of the delivery vehicle formulation. Concentration ranges for vasodilators in the transdermal drug delivery vehicle range from 0.0001% to 2.0% (w/w), preferably less than about 1.0%, depending on the drug to be delivered and also the kinetics of the delivery profile that is desired.

Other elements of the delivery vehicle that need to be empirically identified for the optimal delivery of the drug and also the vasodilators into the skin tissue are the permeation or penetration enhancer molecules. Examples of penetration enhancing substances by example only and not limited to the following list include: natural and complex oils, such as olive, peanut, monoi and sunflower oils to more specific derivatives from the natural oils, such as oleic acid, gamma linoleic acid, stearic acid, lauric acid. Other lipids and phospholipids may also be used to create a microenvironment conducive to the transdermal delivery of drugs, including but not limited to phosphatidylcholine, phosphatidylethanolamine and phosphatidylserine, cholesterol, complexes of phospholipids and other agents to create a formalized structure of liposomes or similar structures designed to facilitate the penetration of the drug delivery vehicle through the skin. Typical concentration ranges for these lipids and fatty acids and oils are between 0.5% to 15%, depending on the characteristics of the penetrating substance and the chemical relationship of these substances with the active drug molecule and the vasodilators.

In addition, either in combination with other penetration enhancing agents or independently, chemicals including isopropanol, propylene glycol, urea, dimethyl acetamide, decylmethyl-sulphoxide, dimethyl-sulphoxide, m-pyrrole, eucalyptus oil, menthol, imidazole as well as other excipients used to serve various roles with different formulations and different drugs designed to facilitate penetration of the active drug molecules and the vasomodulators through the tissue to be treated or through which the drug is to be delivered into the bloodstream.

The active drug molecules that are candidates for transdermal drug delivery defined by this methodology and work and also by the molecular mechanisms governing the transdermal transportation of these drug molecules include but are not limited to the following list of candidate drugs: acetaminophen, acetylsalicylic acid, acyclovir, adrenocorticoids, albuterol, alpha hydroxylipids, aluminum hydroxide, amino acids and amino acid polymers, amoxicillin, androgens, anesthetics, antibody molecules, anticoagulants, antisense molecules, arginine, baclofen, beclomethasone, benzoyl peroxide, betamethasone, botulism toxin, buspirone, caffeine, calcitonin, camptothecin, capsaicin, captopril, carboplatin, cephalexin, cephradine, cetirizine, chloral hydrate, chlorambucil, chloramphenicol, chlorothiazide, chlorotrianisene, chlorpromazine, chlorpropamide, chlorprothixene, chlorthalidone, chlorzoxazone, cholestyramine, cimetidine, cinoxacin, ciprofloxacin, cisapride, cis-platin, clarithromycin, clemastine, clidinium, clindamycin, clofibrate, clomiphere, clonazepam, clonidine, clorazepate, clotrimoxazole, cloxacillin, cloxapine, codeine, colchicine, collagen, coloestipol, conjugated estrogen, contraceptives, corticosterone, cortisone, crornolyn, cyclacillin, cyclandelate, cyclizine, cyclobenzaprine, cyclophosphamide, cyclothiazide, cycrimine, cyproheptadine, cytokines, danazol, darithron, dantrolene, dapsone, daunorubicin, deoxyribonucleic acid, desipramine HCL, desloratidine, desogestrel, dextroamphetamine, dexamethasone, dexchlorpheniramine, dextromethorphan, diazepam, diclofenac sodium, dicloxacillin, dicyclomine, diethylstilbestrol, diflunisal, digitalis, digoxin, diltiazen, dimenhydrinate, dimethindene, diphenhydramine, diphenidol, diphenoxylate & atrophive, diphenylopyraline, dipyradamole, dirithromycin, disopyramide, disulfiram, divalporex, docusate calcium, docusate potassium, docusate sodium, dopamine, domiphen bromide, doxazosin, doxorubicin, doxylamine, dronabinol, enzymes, enalaprilat, enalapril, ephedrine, epinephrine, ergoloidmesylates, ergonovine, ergotamine, erythromycins, erythropoietin, conjugated estrogens, estradiol, estrogen, estrone, estropipute, etbarynic acid, ethchlorvynol, ethinyl estradiol, ethopropazine, ethosaximide, ethotoin, etidronate sodium, etodolac, famotidine, felodipine SR, fenoprofen, fenoterol, fentanyl, ferrous fumarate, ferrous gluconate, ferrous sulfate, fexofenadine, finasteride, flavoxate, flecaimide, fluconazole, fluoxetine, fluphenazine, fluprednisolone, flurazepam, fluticasone, fluticasone propionate, fluvastatin, fluvoxamine maleate, formoterol fumarate, folic acid, fosinopril, furosemide, gabapentin, ganciclovir, gemfibrozil, glimepiride, glipizide, glyburide, glycopyrrolate, gold compounds, gransetron HCl, griseofuwin, growth hormones, guaifenesin, guanabenz acetate, guanadrel, guanethidine, guanfacine, halazepam, haloperidol, heparin, hetacillin, hexobarbital, hydralazine, hydrochlorothiazide, hydrocodone with APAP, hydrocortisone (cortisol), hydroflunethiazide, hydroxychloroquine, hydroxyzine, hyoscyamine, ibuprofen, imipramine, idebenone, indapamide, indomethacin, isradipine, insulin, interferon, ipratropium bromide, iofoquinol, iron-polysaccharide, isoetharine, isoniazid, isopropamide, isoproterenol, isosorbide mononitrate S.A., isotretinoin, isoxsuprine, isradipine, itraconazole, ivermectin, kaolin & pectin, ketoconazole, ketoprofen, ketorolac tromethamine, lactulose, lansoprazole, latanoprost, levodopa, levofloxacin, levonogestrel, levothyroxine, lidocaine, lincomycin, liothyronine, liotrix, lisinopril, lithium, lomefloxacin HCl, loperamide, loracarbef, loratadine, lorazepam, losartan, losartan/HCTZ, lovastatin, loxapine succinate, lymphokines, magnesium hydroxide, magnesium sulfate, magnesium trisilicate, maprotiline, meclizine, meclofenamate, medroxyprogesterone, mefloquine HCl, melatonin, melenamic acid, meloxicam, melphalan, mephenytoin, mephobarbital, meprobanate, mercaptopurine, mesoridazine, metaproterenol, metaxalone, metformin hydrochloride, methadone, methamphetamine, methaqualone, metharbital, methenamine, methicillin, methocarbamol, menthol, methotrexate, methsuximide, methyclothinzide, methylcellulose, methyldopa, methylergonovine, methylphenidate, methylprednisolone, methysergide, methyl salicylate, metformin HCl, metoclopramide, metolazone, metoprolol, metronidazole, mexiletine, miconazole nitrate, minoxidil, misoprostol, mitotane, moclobemide, moexipril HCl, mometasone, monamine oxidase inhibitors, morphine, mupirocin, nabumetone, nadolol, nafazodone, nafcillin, nalidixic acid, naproxen, narcotic analgesics, nedocromil sodium, nefazodone HCl, neomycin, neostigmine, niacin, nicardipine, nicotine, nifedipine, nimodipine, nitazoxanide, nitrates, nitrofurantoin, nitroglycerin, nizatidine, nomifensine, norethindrone, norethindrone acetate, norfloxacin, norgestimate, norgestrel, nylidrin, nystatin, oflaxacin, omeprazole, orphenadrine, oxacillin, oxaprozin, oxazepam, oxprenolol, oxycodone, oxymetazoline, oxyphenbutazone, pancrelipase, pantothenic acid, papaverine, para-aminosalicylic acid, paramethasone, paregoric, paroxetine, pemoline, penicillamine, penicillin, penicillin-v, pentazocine HCl, pentobarbital, pentoxifylline, peptides and peptide fragments, pergolid mesylate, perphenazine, pethidine, phenacetin, phenazopyridine, pheniramine, phenobarbital, phenolphthalein, phenprocoumon, phensuximide, phentolamine mesylate, phenylbutazone, phenylephrin, phenylpropanolamine, phenyl toloxamin, phenytoin, pilocarpine, pindolol, piper acetazine, piroxicum, poloxamer, polycarbophil, calcium, polypeptide fragments, polythiazide, potassium supplements, pravastatin, prazosin, prednisolone, prednisone, primidone, probenecid, probucol, procainamide, procarbazine, prochlorperazine, procyclidine, progesterone, promazine, promethazine, propantheline, propofol, propoxyphene N/APAP, propranolol, proteins and protein fragments, pruzepam, pseudoephedrine, psoralens, psyllium, pyrazinamide, pyridostigmine, pyrodoxine, pyrilamine, pyrvinium, quinapril, quinestrol, quinethazone, quinidine, quinine, rabeprazole, ramipril, ranitidine, rauwolfia alkaloids, riboflavin, ribonucleic acid, rifampicin, risperidone, ritodrine, salicylates, salmeterol, sannosides a & b, scopolamine, secobarbital, senna, serotonin, sertraline, sildenafil citrate, simethicone, simvastatin, sodium bicarbonate, sodium phosphate, sodium fluoride, sodium nitrite, spironolactone, sucrulfate, sulfacytine, sulfamethoxazole, sulfasalazine, sulfinpyrazone, sulfisoxazole, sulindac, sumatriptan, talbutal, tamoxifen, temazepam, tenoxicam, terazosin, terbinafine, terbutaline, terconazole, terfenadine, terphinhydrate, tetracyclines, testosterone and analogs, thiabendazole, thiamine, thioridazine, thiothixene, thonzonium bromide, thyroblobulin, thyroid, thyroxine, tibolone, ticarcillin, timolol, tioconazole, tobramycin, tocainide, tolnaftate, tolazamide, tolbutamide, tolmetin, tramadol, trazodone, tretinoin, triamcinolone, triamterine, triazolam, trichlormethiazide, tricyclic antidepressants, tridhexethyl, trifluoperazine, triflupromazine, trihexyphenidyl, trimeprazine, trimethobenzamine, trimethoprim, trimipramine, tripclennamine, triprolidine, troglitazone, trolamine salicylate, tumor necrosis factor, valacyclovir, valproic acid, valsartan, venlafaxine, verapamil, vitamin A, vitamin B-12, vitamin C, vitamin D, vitamin E, vitamin K, voltarin, warfarin sodium, xanthine, zidovudine, zopiclone, zolpidem.

One or more active ingredients may be used simultaneously or in tandem at the same site or at different sites on the body. The active drug molecules may function in the body as a therapeutic agent to treat a disease of medical condition or serve as a diagnostic tool or agent, or it may serve as a stimulator of other biological processes affecting the health and well being of the body, such as in the case of vaccines and immune reactions. The active ingredient may serve exclusively as the active drug molecule or it may also, in addition, serve as the vasodilator, or the penetrating agent or the binding agent where the active drug molecule exhibits both functions in the drug delivery complex. Typically the concentrations of the active drug molecule ranges from 0.05% to 20% of the delivery vehicle formulation and typically the unit topically-applied dose of the gross formulation is less than 5 grams total for an adult human.

In vivo models have been used to demonstrate the effects of vasodilators on the delivery of drugs from a topical application into the blood. Animal models, which use skin tissue as a regulator of heat and water content typically serve as the best models. Topical drug delivery formulations that incorporate lipids to assist in the passive penetration of the drug molecule through the outer layers of skin in addition to the incorporation of different vasodilators and the therapeutic drug may be used to evaluate the effect of varying levels of vasodilator on the bioavailability of the drug.

### Example 1:

Test formulations containing 15% ibuprofen-sodium salt, 5% oleic acid, 10% menthol, 5% propylene glycol, 10% dimethylacetamide, 1% decylmethylsulfoxide, 1% u-care, varying amounts of tocopherol nicotinate in the range of 0-1%, and 52-53% deionized water were each blended in a beaker with a mechanical mixer and heated to 40°C for 30 minutes until clear, then cooled to room temperature.

150 mg of sodium salt-ibuprofen was formulated with a 1-gram dose of the above lipid-based vehicle formulations containing the increasing amounts of the vasodilator tocopherol nicotinate. The Ibuprofen vehicle was topically applied to rabbits and blood samples were taken over a three-hour period. Plasma was prepared and analyzed for the amount of ibuprofen present in the blood. The data represents the integrated value of ibuprofen concentration in the blood for the three hour time period for each concentration of tocopherol nicotinate.

| Conc. Tocopherol Nicotinate | µg Ibuprofen.hr₍₀₋₃₎.ml⁻¹ |
|---|---|
| Control | 1.03 |
| 0.00010% | 3.98 |
| 0.00025% | 9.48 |
| 0.00050% | 3.12 |
| 0.0010% | 5.08 |
| 0.0100% | 5.84 |
| 0.100% | 4.30 |
| 1.000% | 4.40 |

### Example 2:

Test formulations containing 15% ibuprofen-sodium salt, 5% oleic acid, 10% menthol, 5% propylene glycol, 10% dimethylacetamide, 1% decylmethylsulfoxide, 1% u-care, varying amounts of papaverine ranging from 0-1%, and 52-53% deionized water were each blended in a beaker with a mechanical mixer and heated to 40°C for 30 minutes until clear, then cooled to room temperature.

150 mg of sodium salt-ibuprofen was formulated with the above lipid-based vehicle formulatiosn containing increasing amounts of the vasodilator papaverine. The Ibuprofen vehicle was topically applied to rabbits and blood samples were taken over a three-hour period. Plasma was prepared and analyzed for the amount of ibuprofen present in the blood. The data represents the integrated value of ibuprofen concentration in the blood for the three hour time period for each concentration of papaverine.

| Conc. Papaverine | µg Ibuprofen.hr₍₀₋₃₎.ml⁻¹ |
|---|---|
| Control | 1.03 |
| 0.00010%''' | 5.12 |
| 0.00025% | 4.47 |
| 0.00050% | 8.37 |
| 0.0010% | 7.62 |
| 0.0100% | 5.72 |
| 0.100% | 4.23 |
| 1.000% | 6.16 |

### Example 3:

Maximal blood flow stimulated by the vasodilator tolazoline was measured using a laser Doppler perfusion imager. The maximum blood flow was achieved with a concentration of tolazoline of 0.5%.

Test formulations containing 15% ibuprofen- sodium salt, 5% oleic acid, 10% menthol, 5% propylene glycol, 10% dimethylacetamide, 1% decylmethylsulfoxide, 1% u-care, varying amounts of tolazoline ranging from 0-0.1%, and 52.9-53% deionized water were each blended in a beaker with a mechanical mixer and heated to 40°C for 30 minutes until clear, then cooled to room temperature.

150 mg of sodium salt-ibuprofen was formulated with the above lipid-based vehicle formulatiosn containing increasing amounts of the vasodilator tolazoline. The Ibuprofen vehicle was topically applied to rabbits and blood samples were taken over a three-hour period. Plasma was prepared and analyzed for the amount of ibuprofen present in the blood. The data represents the integrated value of ibuprofen concentration in the blood for the three hour time period for each concentration of tolazoline.

| Conc. tolazoline | µg Ibuprofen.hr₍₀₋₃₎.ml⁻¹ |
|---|---|
| Control | 1.03 |
| 0.0010% | 3.90 |
| 0.0050% | 5.24 |
| 0.0100% | 5.66 |
| 0.0500% | 3.53 |
| 0.100% | 4.53 |

These data indicate that the optical amount of tolazoline is significantly below the amount necessary to stimulate maximum blood flow.

## Claims

1. A method of determining the optimal amount of vasodilator in a topical formulation comprising the vasodilator and an active ingredient, said method comprising:
a. determining the concentration of vasodilator necessary to stimulate maximal dermal blood flow;
b. formulating a first topical formulation with a concentration of vasodilator that is 0.001 times said maximal blood flow concentration;
c. formulating at least a second topical formulation with a vasodilator concentration greater than 0.001 times said maximal blood flow concentration but less than said maximal blood flow concentration;
d. applying said formulations to the skin of an animal; and
e. measuring the amount of said active ingredient present in the blood of said animal as a function of time.

2. The method of claim 1, wherein said second topical formulation has a concentration of vasodilator that is less than the concentration required to stimulate blood flow in said animal.

3. The method of claim 1, further comprising a third formulation having a vasodilator concentration twice that of said first formulation.

4. The method of claim 1, wherein said animal is a human.

5. The method of claim 1, wherein said formulation further comprises a penetration enhancing agent.

6. The method of claim 1, wherein said step of determining the maximal dermal blood flow comprises applying said vasodilator to the skin, and measuring the blood flow with a laser Doppler perfusion imager.
